(19) Europäisches Patentamt / European Patent Office / Office européen des brevets

(11) **EP 4 122 535 B1**

(12) **EUROPEAN PATENT SPECIFICATION**

(45) Date of publication and mention
of the grant of the patent:
**03.09.2025 Bulletin 2025/36**

(21) Application number: **22185763.4**

(22) Date of filing: **19.07.2022**

(51) International Patent Classification (IPC):
*A61N 5/10* *(2006.01)*      *G01T 1/185* *(2006.01)*

(52) Cooperative Patent Classification (CPC):
**A61N 5/1048; G01T 1/185**

(54) **METHOD FOR CONTROLLING THE RADIOTHERAPY TREATMENT OF CANCER PATIENTS AND RELATED CONTROL DEVICE**

VERFAHREN ZUR STEUERUNG DER STRAHLENTHERAPIEBEHANDLUNG VON KREBSPATIENTEN UND ZUGEHÖRIGE STEUERVORRICHTUNG

PROCÉDÉ DE COMMANDE DE TRAITEMENT PAR RADIOTHÉRAPIE DE PATIENTS ATTEINTS DE CANCER ET DISPOSITIF DE COMMANDE ASSOCIÉ

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **22.07.2021 IT 202100019520**

(43) Date of publication of application:
**25.01.2023 Bulletin 2023/04**

(73) Proprietor: **S.I.T. - Sordina IORT Technologies S.p.A.**
**36100 Vicenza (VI) (IT)**

(72) Inventors:
• **DI MARTINO, Fabio**
 **36100 Vicenza (IT)**
• **FELICI, Giuseppe**
 **36100 Vicenza (IT)**
• **DI FRANCESCO, Massimo**
 **36100 Vicenza (IT)**
• **GALASSO, Vincenzo**
 **36100 Vicenza (IT)**
• **BARONE, Salvatore**
 **36100 Vicenza (IT)**

(74) Representative: **Burchielli, Riccardo et al**
**Barzano & Zanardo S.p.A.**
**Via Piemonte 26**
**00187 Roma (IT)**

(56) References cited:
WO-A1-2014/065990    WO-A1-2021/050535
DE-A1- 19 907 207    US-A1- 2017 350 991

**Description**

The present invention concerns a method for finding and setting the parameters for a device, called "ALLS" gas chamber, for the radiotherapy treatment of cancer patients.

The invention also concerns an ionisation chamber, which allows to work and, therefore, compatible with "Flash" radiotherapy regimes involving a much higher dose rate to the target than the standard case, which will be explained in detail later.

It is known that radiotherapy is a consolidated instrument for treating cancer patients and together with surgery and chemotherapy it has significantly improved both the final prognosis and the quality of life of patients.

The effectiveness associated with the radiotherapy treatment lies in the possibility of hitting diseased cells with a dose of radiation high enough to be curative, limiting the damage to the surrounding healthy tissues.

To carry out an effective treatment, several strategies are implemented:

a) Optimisation of beam ballistics: ionising radiations and different technologies are chosen according to the tumor to be treated, using, with external beams, several input fields; in this way, "conformed" dose distributions are obtained on the target to be treated, with a dosimetric saving of adjacent healthy tissues.

b) Optimisation of radiobiological parameters: where possible, the radiobiological fact resulting from a better and earlier recovery of healthy tissues from sub-lethal radiation damage at cellular level, compared to diseased tissues, is exploited. In this way it is possible to be very effective on the tumor limiting damage to healthy tissues, by splitting the treatment dose. In fact, for example, a standard radiotherapy technique is represented by a treatment splitted in a series of treatments of 2 Gy per day, to be repeated until the prescribed dose (60 - 80 Gy) is reached.

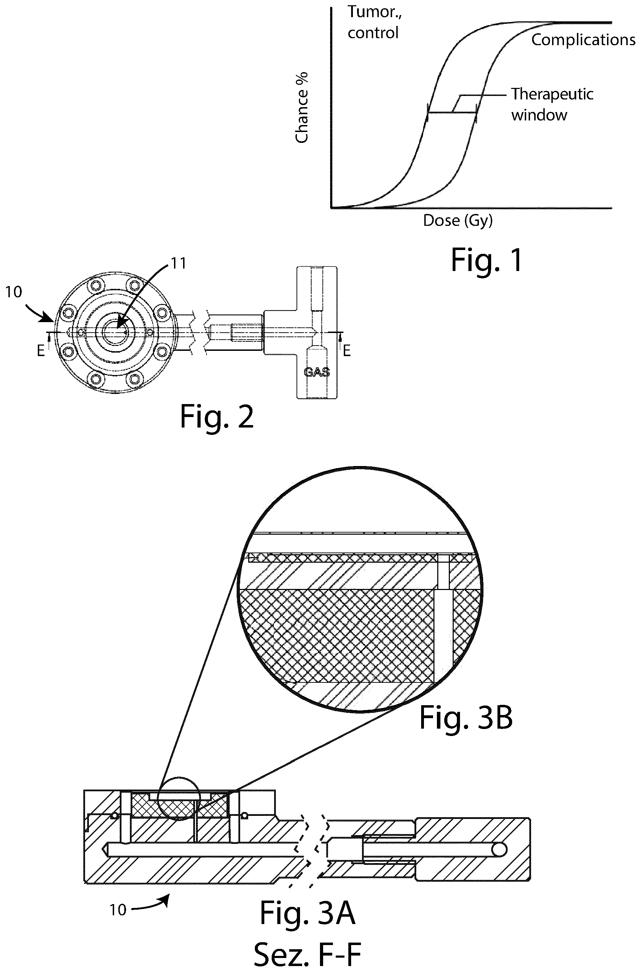

Ultimately, the effectiveness of a radiotherapy treatment results from the possibility of having a "therapeutic window" (in this regard, see the diagram in the attached Figure 1, wherein the "therapeutic window" is defined as the distance between the curves) between the curative dose to the target (upper curve of Figure 1) and the damage to healthy tissue (lower curve of Figure 1).

The prognosis is closely related to the existence of such "therapeutic window"; tumors with a more complex prognosis are those where it is complicated, if not impossible, to deliver effective doses to the target (good chance of recovery) without having limiting complications.

Consider, by way of example but not limited to, pancreatic cancer, which today has a very poor prognosis, characterised by a five-year survival lower than 20%, even in the most favorable cases.

The treatment of these difficult tumours, which have resisted the massive technological development in this sector since the 80s, requires new ideas, techniques and discoveries.

Radiotherapy is currently administered through multiple devices: radioisotopes, electron and/or X-ray linear accelerators, proton and/or carbon ion cyclotrons.

However, all these technologies share a variable dose rate but substantially limited to a few Gy per minute.

Some researches have shown an unexpected and potentially revolutionary phenomenon: using a much higher dose rate, defined as the "Flash" effect, and equal to about 40 Gy/s, higher than the standard by a factor greater than 1000, the ratio between the damage to diseased cells compared to healthy tissue was no longer that obtainable with standard dose rates, but much more advantageous. For example, it was possible to completely cure lung tumour in guinea pigs without the side effects of the standard technique.

Through these researches it has been shown that in the "Flash" range the so-called "therapeutic window" (see diagram in Figure 1) was significantly greater, suggesting a higher chance of a positive prognosis for tumors not yet curable, thus making curable what is not curable today, and significantly improving the quality of life of cancer patients.

One of the main problems that limits adoption of radiotherapy in "Flash" mode is the absence of equipment and methods that allow the beam real-time measurement where there are doses per pulse and dose rates significantly higher than today's standards.

In particular, doses higher than today's standards typically mean doses of the order of a few cGy/pulses and rates of the order of tens of Gray/minute, when instead a radiation beam, in order to generate the 'Flash' effect requires a dose per pulse and higher dose rates, respectively, by at least a factor of 100 and 10000.

In fact, the ionisation chambers, the main instrument in the commissioning of medical accelerators, are currently unusable.

The classic formula ("Absorbed Dose Determination in External Beam Radiotherapy", Technical Report Series No. 398 (TRS 398)) for calculating the dose with an ionisation chamber is as follows :

$$D_W = N_{D,W} \, M_k \, k_{sat} \, k_q$$

where Dw is the dose measured in water, $N_{D,W}$ is the chamber calibration factor, $M_k$ is the correct charge reading for temperature and pressure, $k_{sat}$ is the factor that takes into account ionic recombination, $k_q$ is the factor correcting the reading for the different type of beam quality compared to calibration conditions.

This approach is clearly not applicable because in this case the $k_{sat}$ factor, taking into account the different ionic recombination with the beam under measurement compared to the known calibration conditions, cannot be in any way determined and in any case the physics of the process cannot be in any way attributed to the modelisation behind "TRS 398".

In the design of an ionisation chamber in the "Flash" regime, it is necessary that reading remain proportional to the accumulated dose, i.e. that no saturation phenomena occur such as to reduce or cancel the sensitivity of the chamber itself.

There are two different phenomena that need to be controlled and solved:

- The ionic recombination;
- The generation of an electric field by the ionised charges such as to cance out or reverse the electric field generated by the external polarisation of the chamber. It is crucial that the electric field does not cancel out because if this were to happen, a direct recombination between electrons and positive ions would occur.

Furthermore, it is necessary to ensure that the chamber, once the value of the polarisation voltage has been fixed, maintains the same proportionality factor with the dose absorbed as the dose per pulse varies. To obtain what has just been described, it is necessary to make sure that you are never in the geiger regime, i.e. in the production regime of uncontrolled "discharges", and that the perturbation of the electric field generated by the charges per pulse, produced inside the chamber, is negligible compared to the static field applied between the electrodes (V/d).

The solutions that have been proposed to date, to overcome the issues indicated above, use:

1. the reduction of the distance between the electrodes in order to reduce the flight time of the charges before their collection on the electrode;
2. noble gases that are not subject to ionic recombination.

In detail, the first solution is limited, not only by technical manufacturing difficulties and by the mechanical stability of a chamber with interelectrode distances of the order of 0.1 mm, but especially by the conceptual limit given by the discharge threshold. In fact, it can be shown that such solution does not allow to measure doses per pulse higher than 10 Gy/p.

With reference to the second solution, without an adequate design and a specific calculation of the relevant parameters, it does not solve the issue of cancelling out the electric field nor does it guarantee to work in an ionisation chamber regime or at least, where working in a proportional regime, to maintain constant the production of secondary ions as the dose per pulse varies.

A method for finding and setting the parameters for a gas chamber appropriately for a given dose per pulse for a radiotherapy treatment of cancer patients having the features of the preamble of the appended claim 1 is known for example from WO2014/065990A1 and WO2021/050535A1.

The scope of the present invention is defined by the claims which follow.

The main aim of the present invention is therefore to realise a method for finding and setting the parameters for a gas chamber for a radiotherapy treatment of cancer patients, which is such as to overcome the issues of the known art.

A further aim of the present invention is to realise a method for finding and setting the parameters for a gas chamber for aradiotherapy treatment of cancer patients that is able to operate in "Flash" mode, a mode where over 10 Gy/p are reached, i.e. three orders of quantity of absorbed dose greater than traditional linear accelerators.

Another aim of the present invention is to realise a method for finding and setting the parameters for a gas chamber for a radiotherapy treatment of cancer patients, whose measurements are accurate, despite the work regime.

The aim of the present invention is also to realise a method for finding and setting the parameters for a gas chamber for a radiotherapy treatment of cancer patients, which allow the dose measurement with a direct reading device thus solving the issue of dosimetry by means of a gas chamber, only by way of example a gas chamber working in an ionising regime, of any "Flash" beam with electrons.

Last but not the least aim of the present invention is to realise a method for finding and setting the parameters for a gas chamber for a radiotherapy treatment of cancer patients which is such as to control and solve phenomena such as ionic recombination and generation of an electric field by the ionised charges such as to cancel out or reverse the electric field generated by the external polarisation of the chamber.

These and other aims are achieved by a method according to the attached claim 1; other detailed technical characteristics are contained in the subsequent claims.

Another object of the present invention is a method wherein the reading remains proportional to the accumulated dose, i.e. phenomena of alteration of the electric field such as to reduce or cancel the sensitivity of the chamber itself (saturation), and/or uncontrolled production of secondary ionisations (which would cause the proportionality loss) do not occur.

These and other aims are achieved by a method for finding and setting the parameters for a gas chamber for a radiotherapy treatment of cancer patients, as it will become clearer in the rest of the present description, according to a preferred but not exclusive embodiment of the present invention and with reference to the attached sheets of drawings, in which:

Figure 1 schematically shows a diagram relating to the known concept of "therapeutic window" in radiotherapy;
Figure 2 schematically shows a top view of a device used for the radiotherapy treatment of cancer patients, according to the present invention;
Figure 3A schematically shows a side view in longitudinal section of the device of Figure 2, according to the present invention;
Figure 3B shows an enlargement of a detail of the device of Figure 2;
Figure 4 relates to an exploded view of the device of Figure 2;
Figure 5 relates to a perspective view of the device of Figure 2, according to the present invention;
Figure 6 relates to the operation reference system of the device of Figure 2, according to the present invention;
Figure 7 schematically shows the typical trend of the electric field generated by ions with respect to the distance $d$ from a positive electrode;
Figure 8 schematically shows the temporal dynamics of the charge density;
Figure 9 schematically shows the trend of the so-called "Paschen Curve" for the Argon gas with which the device of Figure 2 is filled, according to the present invention.

The FLASH work regime, which requires doses per pulse typically equal to or greater than 1 Gy/p, does not allow to use the standard Boag theory, previously described, on the estimation of the correction factor $k_{sat}$ for the incomplete collection of the charge generated by the pulse in the chamber. In fact, it is not possible to ignore the effect of the field generated by the moving charges after ionisation.

Starting from these considerations, and with reference to Figures 2-5, the device, object of the present invention, comprises a gas chamber 10, only by way of example a gas chamber which works in ionisation regime, with flat and parallel electrodes 7, which is designed in such a way that there is a distance d between electrodes.

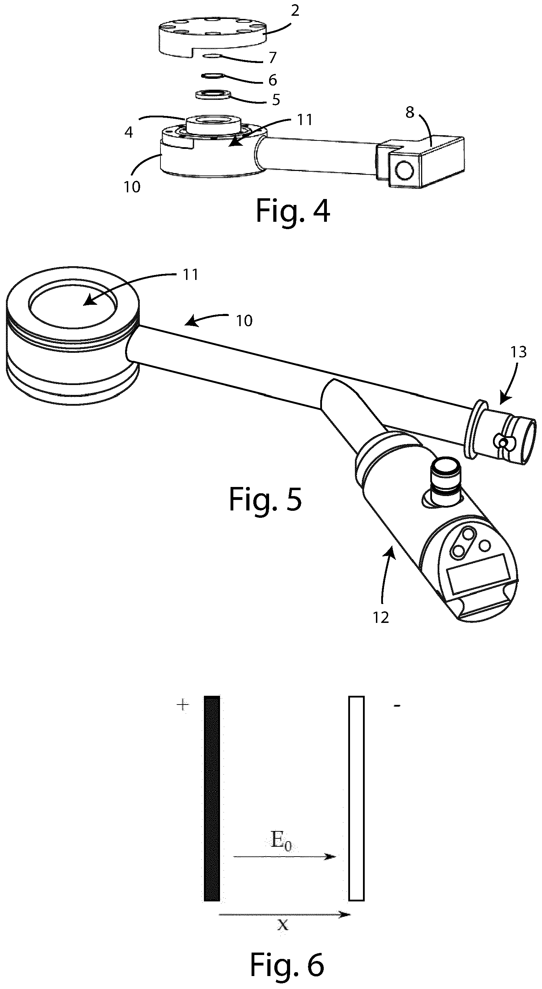

Furthermore, as it can be seen in Figure 4, the device also comprises a window 2 placed above the aforementioned electrode 7. Advantageously, there are a first insulator 6, a guard element 5 and, finally, a second insulator 4, placed below the electrode 7, opposite to the window 2. Finally, again advantageously, connected to the chamber 10 through, for example, a pipe, there is a collector 8, in particular for the noble gas to be inserted inside the cavity 11. Again advantageously, at the collector 8 there is a pressure gauger 12 for controlling the pressure P and, in preferred embodiments, a triaxial connector 13 suitable for ultra high vacuum (UHV).

Advantageously, to avoid a priori the formation of negative ions in the chamber 10, the gas chamber 10 comprises a cavity 11 which is filled with a noble gas since, again advantageously, this type of gas does not have negative ions that are generated following the ionisation event. In particular, in a preferred but not limiting embodiment, the noble gas that is used is Argon, but any other noble gas could be used.

In this condition the electrons generated can be considered as free and, since their mobility is much higher than that of positive ions (by a factor ~$10^3$), their direct recombination is considered negligible as long as they are subjected to a non-zero electric field (for example, facing the positive electrode of the chamber).

As regards the electric field inside the chamber, we distinguish two contributions: that one due to the polarisation of the chamber and that one due to the charges generated by the radiation pulse. The polarisation field of the chamber faces the positive electrode, it can be considered uniform and constant in the chamber volume; we indicate it as $\vec{E}_0$ (Figure 6).

To calculate the generated field of the released charges we analyse the temporal dynamics of the physical processes. We hypothesise that an instant after the radiation pulse the generated charges have led to two uniform charge distributions. Given the great difference in mobility, the electron migration occurs in a much shorter time than that of positive ions: we can consider stationary ions and electrons in motion. Where there is the "overlap" between electrons and ions, the net charge density is zero, so instantly after the pulse the electrons will move under the influence of the field $\vec{E}_0$, therefore towards the positive electrode. The "overlap" zone will shrink over time, due to the collection of free electrons. Our objective is to describe the field in the worst condition as regards the collection of electrons: that one for which the "overlap" zone does not exist and only a uniform distribution of positive ions in the volume of the chamber 10 is considered.

An expression for the field of positive ions $\vec{E}_{ION}$, facing the negative electrode of the chamber, is obtained starting from the Poisson equation:

$$\frac{d^2V}{dx^2} = -\frac{q_V}{\epsilon_0 \epsilon_r}$$

By integrating and exploiting the fact that at d/2 the field (E = *-dV/dx*) must be equal to 0 for symmetry, we obtain:

$$E_{ION}(t) = \frac{q_V}{\epsilon_0 \epsilon_r}\left(x - \frac{d}{2}\right)$$

Where $q_V$ is the volumetric charge density in the chamber volume.

The total field inside the chamber is obtained from the overlap principle:

$$\vec{E} = \vec{E}_0 + \vec{E}_{ION}$$

We want this field to always face the positive electrode, so for the chosen reference system, we want E > 0 and to ensure that this condition occurs it will be sufficient that E(0) > 0. We thus obtain:

$$E_0 + E_{ION}(0) > 0$$

$$E_0 > \frac{q_V}{\epsilon_0 \epsilon_r}\frac{d}{2}$$

To respect the boundary condition of the potential on the chamber electrodes, the following also applies:

$$\int_0^d \vec{E} \cdot d\vec{x} = \int_0^d E_0 dx + \int_0^d E_{ION} dx = V$$

$$E_0 = \frac{V}{d}$$

From which it is immediately derived the voltage limit value to be applied to have a field always facing the positive electrode and thus avoid the recombination of electrons:

$$V_{LIM} = \frac{q_V}{\epsilon_0 \epsilon_r}\frac{d^2}{2}$$

The charge density of the positive ions generated in the gas chamber depends on the radiation pulse (which refers to the dose in water) $D_p$:

$$q_V^{gen} = \frac{D_p \cdot \overline{S}_W^{GAS} \cdot k_Q \cdot \varrho}{w/e}$$

Where $\overline{S}_W^{GAS}$ is the ratio of the stopping power of the gas to that of water, ρ is the gas density in the chamber and kQ is the ratio between the reading of the chamber in calibration conditions and in working conditions (as per the TRS 398 formalism).

The limit voltage, using the generated charge, is therefore:

$$V_{LIM} = \frac{D_p \cdot \overline{S}_W^{GAS} \cdot k_Q \cdot \varrho}{2(w/e)\epsilon_0\epsilon_r} \cdot d^2$$

The value of $V_{LIM}$ calculated above is in fact an overestimate because the maximum charge will be lower due to the depletion effect discussed in the next paragraph.

The estimate previously obtained for the limit voltage is an overestimate as it does not take into account the temporal dynamics of the processes we are analysing. In particular, it does not take into account the collection of positive ions during the time of the radiation pulse of duration T. This collection decreases the charge generating the perturbative field and consequently decreases the minimum voltage necessary to avoid the recombination of the electrons.

Assuming that the dose deposition is linear over time, we can write its expression as:

$$D(t) = \frac{D_p \cdot t}{T}$$

At this point at a time instant $dt$ we will have a part of the charge $dQ^{gen}$ generated by the radiation and a part of the charge $dQ^{rac}$ collected by the electrodes of the chamber. The net infinitesimal charge is:

$$dQ = dQ^{gen} - dQ^{rac}$$

The charge density generated at the time instant depends on the deposition of the radiation pulse:

$$\dot{q}_V{}^{gen} = \frac{1}{V}\frac{dQ^{gen}}{dt} = \frac{\dot{D}(t) \cdot \overline{S}_W^{GAS} \cdot k_Q \cdot \rho \cdot (\rho/\rho_0)}{w/e} = \frac{D_p \cdot \overline{S}_W^{GAS} \cdot k_Q \cdot \varrho}{T \cdot w/e}$$

Where V is the chamber volume and therefore qv = Q/V.

While the charge collected by the electrodes at the time instant is:

$$\delta Q^{rac} = q_V \cdot \delta V$$

$$\delta Q^{rac} = q_V \cdot S \cdot \delta x$$

$$\dot{q}_V{}^{rac} = \frac{1}{V}\frac{dQ^{rac}}{dt} = \frac{q_V \cdot dx \cdot S}{V dt} = \frac{q_V \cdot v_D}{d}$$

Where $v_D$ is the drift velocity of the positive ions which is linked to mobility and to the electric field by $v_D = \mu E$. It is important to note how the mobility itself is inversely proportional to the gas density in the chamber, by indicating with $\rho_0$ the gas density at atmospheric pressure, with $\mu_0$ the mobility at atmospheric pressure and with $\rho$ the gas density at a certain pressure we have:

$$v_D = \mu_0 \frac{\rho_0}{\rho} E$$

The expression for the collected volumetric charge density therefore becomes:

$$\dot{q}_V{}^{rac} = \frac{q_V \cdot \mu_0 \cdot (\rho_0/\rho) \cdot E}{d}$$

Since the collection of ions occurs at the negative electrode, the field to be considered will be $E(d)$ i.e. the maximum field:

$$\dot{q_V}^{rac} = \frac{q_V \cdot \mu_0 \cdot (\rho_0/\rho)}{d} \cdot (\frac{V}{d} + \frac{q_V}{2\epsilon_0\epsilon_r}d)$$

By putting the various contributions together, the net charge rate in the chamber is obtained:

$$\dot{q_V} = \frac{D_p \cdot \overline{S_W}^{GAS} \cdot k_Q \cdot \rho \cdot (\rho/\rho_0)}{T \cdot w/e} - \frac{\mu_0 \cdot (\rho_0/\rho)}{2\epsilon_0\epsilon_r} \cdot q_V^2 - \frac{\mu_0 \cdot (\rho_0/\rho) \cdot V}{d^2} \cdot q_V$$

Which, for convenience, we can rewrite as:

$$\dot{q_V} = Aq_V^2 + Bq_V + C$$

where

$$\begin{cases} A = & -\dfrac{\mu_0 \cdot (\rho_0/\rho)}{2\epsilon_0\epsilon_r} \\[2ex] B = & -\dfrac{\mu_0 \cdot (\rho_0/\rho) \cdot V}{d^2} \\[2ex] C = & \dfrac{D_p \cdot \overline{S_W}^{GAS} \cdot k_Q \cdot \rho \cdot (\rho/\rho_0)}{T \cdot w/e} \end{cases}$$

The differential equation to be solved is of the type:

$$\frac{dy}{dx} = Ay^2 + By + C$$

The singular solutions are found by setting:

$$Ay^2 + By + C = 0$$

Removed the singular solutions, we have an equation with separable variables that can be rewritten:

$$\frac{dy}{Ay^2 + By + C} = dx$$

We integrate both sides, the integration extremes of x are between *[0,* T] and the boundary condition is that y(0) = 0.

We obtain, being $\Delta = B^2 - 4AC > 0$:

$$\int_{y=0}^{y(x)} \frac{dy}{Ay^2 + By + C} = -\frac{2}{\sqrt{\Delta}}\text{arctanh}(\frac{2Ay + B}{\sqrt{\Delta}})\Big|_{y=0}^{y(x)}$$

$$= -\frac{2}{\sqrt{\Delta}}\text{arctanh}(\frac{2Ay(x) + B}{\sqrt{\Delta}}) + \underbrace{\frac{2}{\sqrt{\Delta}}\text{arctanh}(\frac{B}{\sqrt{\Delta}})}_{=k}$$

The right one instead:

$$\int_{s=0}^{x} ds = x$$

Solving the equation for y(x) we have:

$$y(x) = \frac{\sqrt{\Delta}\,tanh(\frac{\sqrt{\Delta}}{2}(k - x)) - B}{2A}$$

This equation allows to analytically evaluate the maximum charge density actually present and therefore its maximum perturbative effect on the electric field.

This evaluation allows to:

- check that the field does not cancel out;
- evaluate the maximum accuracy obtainable for the desired dose per pulse value.

By way of numerical example, we want to measure a dose per pulse equal to 40 Gy; the operating values to perform the measurement are shown in the following table

| Quantity | Value | Unit of measurement |
|---|---|---|
| Distance between electrodes d | 1 | mm |
| Argon density STP $\rho_0$ | 1.78 | kg/m$^3$ |
| Dose per pulse $D_p$ | 40 | Gy = J/kg |
| Pulse duration T | 4 | $\mu$s |
| Argon pair production energy w/e | 26 | eV = J/C |
| Argon mobility STP$^2$ $\mu_0$ | 1.5 · 10$^{-4}$ | m$^2$/(Vs) |
| **Dielectric** constant of Argon $\varepsilon_0\varepsilon_r$ | 8.85 · 10$^{-12}$ | C/(Vm) |

These therefore represent the operating conditions suitable for measuring a dose per pulse equal to 40 Gy/p.

Advantageously, in order to be able to use the gas chamber 10 as a detector of the deposited dose, it is essential to avoid the Geiger regime for the chamber, a regime where there is an uncontrolled generation of the secondary ionisation with the deposited dose. Therefore it becomes essential being below the breakdown voltage $V_b$, after which the aforementioned Geiger regime is entered.

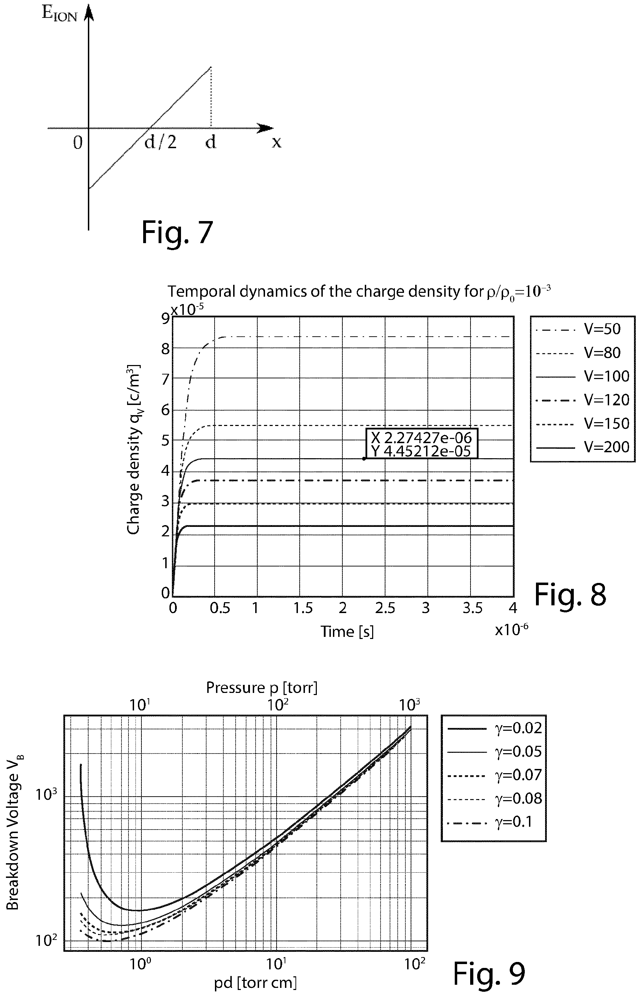

The trend of the breakdown voltage $V_b$, as the distance between the electrodes of the chamber 10 and the pressure of the gas inside the chamber vary, is described by the trend of the Paschen curve shown in Figure 8: it is always possible to vary the pressure so as to avoid such regime.

For example, in the numerical case analysed above, with $\rho/\rho_0$ = 10$^{-3}$, for a polarisation field V = 100V we are well below the breakdown voltage $V_b$.

Even after all the precautions of the previous sections, we don't know if the operating regime is that of an ionisation chamber (i.e. absence of production of secondaries) or of the proportional regime. The chance of generating secondaries is in fact linked to the energy acquired inside the charge by ions which is proportional to the electric field (and therefore to the polarisation voltage).

In the proportional regime operation, the fundamental aspect is the stability of the proportionality factor between the charge collected and the dose deposited in the chamber, this stability exists only when the number of secondary products remains almost constant as the dose per pulse varies. In view of the above, this situation occurs when the electric field due to the accumulation of the charge $E_{ION}$ is negligible compared to the polarisation electric field of the chamber E0. Practically it must be evaluated the ratio:

$$\frac{\Delta E}{E} = \frac{E_{ION}(d)}{E_0} = \frac{q_V \cdot d^2}{2\epsilon_0 \cdot \epsilon_r \cdot V}$$

Which, given $\rho/\rho_0$ = 10$^{-3}$ and a polarisation V = 100V, has a value of about 2.5%.

However, this is a very conservative calculation because the highest value of the field generated by ions has been considered, i.e. the one at the edge: the average perturbation effect will in any case be lower.

On the basis of what has been said, advantageously, the cavity 11 of the chamber 10 is filled with gas with an adjustable pressure depending on the maximum dose per pulse to be measured (see the equations of the previous paragraph).

Furthermore, the implementation of such solutions requires:

- The use of materials for the body of the chamber 10 suitable for sustaining an internal pressure lower than atmospheric pressure, for example, a condition of depression up to $10^{-1}$ mbar, which allows to measure up to 500 Gy/pulse;
- The use of braze-welding techniques for making the chamber body in order to maintain the aforementioned depressions;
- The use of a pressure switch and of a needle valve for adjusting pressure.

Advantageously, the charge collected by the gas chamber 10 is read by means of a suitable electrometer, for example of a known type, with a bias voltage varying between $\pm$ 400 V.

Advantageously, the operational scheme of the method for controlling the radiotherapy treatment consists of the following steps:

A. the maximum dose per pulse value to be measured is fixed, for example 40 Gy/pulse;

B. Calculation of the charge density per unit volume (Qv) generated by the pulse inside the chamber 10;

C. Calculation of $V_{lim}$, i.e. the minimum value of the voltage V applied to the electrodes 7, necessary so that the field Q never cancels out during the collection of electrons, thus avoiding the direct recombination thereof with ions. In this way, any type of recombination is avoided, because the positive ions do not recombine (noble gas);

D. Fixing a voltage value V (for example 100 V) to calculate $\rho$, the gas density and, consequently P (pressure) in such a way that $V>V_{lim}$ (the voltage greater than the minimum value of the voltage V applied to the electrodes 7);

E. Analysis of the Paschen curve thus carried out, to check that the pair of values $\rho$/V (density/voltage) is in a point of the curve that guarantees to be outside the Geiger regime (uncontrolled production of secondary ionisations).
If this were not the case, there would be an additional step, for example E1, wherein $\rho$ would be decreased accordingly, choosing the new density value in order to exit this regime.

F. Insertion of the temporal dynamics of positive ions and calculation **of** $Qv_{MAX}$, i.e. the maximum charge density present during the entire duration of the pulse, which is obviously less than the total QV generated.

G. Fixing the maximum acceptable inaccuracy value on the measurement, caused by the electric field perturbation over the whole range of dose per pulse to be investigated ($\Delta$E/E).

For example, if this value is 3%; it is calculated if this accuracy is reached with the value of $Qv_{MAX}$ fixed in the previous step F; if the answer is affirmative, the parameters of chamber 10 fixed in this way are optimal, vice versa, if the answer is negative, $\rho$ (the density) is further decreased and we start again from the previous Step F.

Finally, to summarise what has been said so far:

1) by using a noble gas, the ionic recombination is avoided and, by ensuring that the field never cancels out during the collection of electrons, the ion/electron recombination is also avoided

2) by fixing the voltage V and decreasing the gas pressure P it is obtained:

2.1) the certainty of being outside the uncontrolled Geiger regime (Paschen curve, Figure 9);
2.2) making sure that the field never cancels out up to a fixed dose per pulse value.

Having fixed V and decreased the pressure to obtain the above, this leads to a parallel increase of the drift velocity of the charges with a high chance of generating secondary ionisations. This means that, with great probability, we are no longer in a noble gas chamber regime but in a proportional regime.

However, to use the chamber filled with noble gas as a dosimeter, it is necessary to be sure that the proportionality regime is constant as the dose per pulse varies in the range of our interest within a desired accuracy. This is always obtained by acting on gas pressure P, since by decreasing the pressure and increasing the drift velocity of charges, a "depletion effect" of the charge inside the chamber 10 is generated during the pulse. Advantageously, this causes the maximum accumulated charge to be less than that generated and, consequently, its perturbative effect on the electric field and the production of secondary ionisations.

The theory analytically formalises these concepts and, by fixing the maximum dose per pulse and the accuracy with which it is intended to measure, allows to calculate the parameters of the chamber 10 meeting these requirements.

**Claims**

1.  Method for finding and setting the parameters for a gas chamber for a radiotherapy treatment of cancer patients wherein a gas chamber (10) with flat and parallel electrodes (7) is provided, said electrodes (7) being placed at a given distance (d), a window (2) being placed superiorly to an electrode (7) and insulating means (4, 5, 6) being placed inferiorly to said electrode (7), wherein said gas chamber (10) is connected to a collector (8) by means of which a noble gas, preferably Argon, is inserted inside a cavity (11) of said chamber (10) with an adjustable pressure depending on the maximum dose per pulse to be measured, so that the electric field inside said chamber (10) is due to the polarisation of said chamber (10) and to the charges generated by a radiation pulse,
    **characterised in that** said method provides the following steps:

    - determination of a maximum dose per pulse value to be measured;
    - calculation of the charge density per unit volume generated by the pulse inside said gas chamber (10);
    - calculation of a minimum voltage value applied to said electrodes (7) necessary so that the electric field never cancels out during the collection of electrons;
    - determination of a voltage value, in order to calculate the density and pressure of the noble gas, in such a way that said voltage value is greater than the minimum voltage value applied to said electrodes (7);
    - analysis and check that the pair of density and voltage values are outside the Geiger regime and possible decrease of the density value in case said pair of values is inside the Geiger regime, choosing the new density value in order to exit from such regime;
    - insertion of a temporal dynamics of positive ions and calculation of the maximum charge density present during the entire duration of the pulse;
    - determination of a maximum acceptable value of inaccuracy on the measurement, caused by the perturbation of the electric field over the range of dose per pulse to be detected, and possible decrease of the density value in case this maximum value is exceeded.

2.  Method as in claim 1 **characterised in that**, said voltage value being fixed, said noble gas pressure is decreased in order to be outside the Geiger regime and in order to ensure that the electric field never cancels out up to a fixed dose per pulse value.

3.  Method as in claim 2, **characterised in that** said decrease in pressure and an increase in charge drift velocity generates during the pulse a charge depletion effect within the gas chamber (10), so that the maximum accumulated charge is less than the generated charge and, consequently, its perturbative effect on the electric field and the production of secondary ionisations is lower.

4.  Method as in claim 1, **characterised in that** at said collector (8) there is a pressure gauge (12) for controlling the pressure (P) of said noble gas.

5.  Method as in claim 1, **characterised in that** at said collector (8) there is a triaxial connector (13) adapted to realise ultra-high vacuum (UHV) conditions.

6.  Method as in claim 1, **characterised in that** said gas chamber (10) is made of materials adapted to sustain internal pressure values lower than atmospheric pressure and using braze-welding techniques in order to maintain said internal pressure values.

7.  Method as in claim 1, **characterised in that** it comprises an electrometer having a variable bias voltage, adapted to measure the charge collected by said gas chamber (10).

**Patentansprüche**

1.  Verfahren zum Finden und Einstellen der Parameter für eine Gaskammer für eine Strahlentherapiebehandlung von Krebspatienten, bei dem eine Gaskammer (10) mit flachen und parallelen Elektroden (7) vorgesehen ist, wobei die Elektroden (7) in einem gegebenen Abstand (d) angeordnet sind, ein Fenster (2) oberhalb einer Elektrode (7) angeordnet ist und Isoliermittel (4, 5, 6) unterhalb der Elektrode (7) angeordnet sind, **dadurch gekennzeichnet, dass** die Gaskammer (10) mit einem Kollektor (8) verbunden ist, durch den ein Edelgas, vorzugsweise Argon, in einen Hohlraum (11) der Kammer (10) mit einem einstellbaren Druck in Abhängigkeit von der zu messenden maximalen Dosis pro Impuls eingeleitet wird, so dass das elektrische Feld im Inneren der Kammer (10) auf die Polarisation der Kammer (10) und auf die durch einen Strahlungsimpuls erzeugten Ladungen zurückzuführen ist, **dadurch gekennzeichnet, dass** das Verfahren die folgenden Schritte umfasst:

- Bestimmung eines maximalen Dosiswertes pro Impuls, der gemessen werden soll;
- Berechnung der Ladungsdichte pro Volumeneinheit, die durch den Impuls in der Gaskammer (10) erzeugt wird;
- Berechnung eines Mindestwertes für die an die Elektroden (7) angelegte Spannung, der erforderlich ist, damit sich das elektrische Feld während des Auffangens der Elektronen nicht aufhebt;
- Bestimmung eines Spannungswertes, um die Dichte und den Druck des Edelgases zu berechnen, so dass der Spannungswert größer ist als der minimale Spannungswert, der an die Elektroden (7) angelegt wird;
- Analyse und Überprüfung, ob das Paar von Dichte- und Spannungswerten außerhalb des Geiger-Regimes liegt, und mögliche Verringerung des Dichtewerts, falls das besagte Wertepaar innerhalb des Geiger-Regimes liegt, wobei der neue Dichtewert gewählt wird, um dieses Regime zu verlassen;
- Einfügen einer zeitlichen Dynamik positiver Ionen und Berechnung der maximalen Ladungsdichte während der gesamten Dauer des Pulses;
- Bestimmung eines maximal akzeptablen Wertes für die Ungenauigkeit der Messung, die durch die Störung des elektrischen Feldes über den Bereich der zu erfassenden Dosis pro Impuls verursacht wird, und mögliche Verringerung des Dichtewertes, falls dieser Maximalwert überschritten wird.

2. Verfahren nach Anspruch 1, **dadurch gekennzeichnet, dass** bei festem Spannungswert der Edelgasdruck verringert wird, um außerhalb des Geiger-Regimes zu sein und um sicherzustellen, dass sich das elektrische Feld niemals bis zu einem festen Dosiswert pro Impuls aufhebt.

3. Verfahren nach Anspruch 2, **dadurch gekennzeichnet, dass** die Abnahme des Drucks und eine Zunahme der Ladungsdriftgeschwindigkeit während des Pulses einen Ladungsverarmungseffekt innerhalb der Gaskammer (10) erzeugt, so dass die maximale akkumulierte Ladung geringer ist als die erzeugte Ladung und folglich ihre störende Wirkung auf das elektrische Feld und die Erzeugung von Sekundärionisationen geringer ist.

4. Verfahren nach Anspruch 1, **dadurch gekennzeichnet, dass** an dem Kollektor (8) ein Druckmesser (12) zur Kontrolle des Drucks (P) des Edelgases vorhanden ist.

5. Verfahren nach Anspruch 1, **dadurch gekennzeichnet, dass** sich an dem Kollektor (8) ein triaxialer Anschluss (13) befindet, der geeignet ist, Ultrahochvakuum (UHV)-Bedingungen zu realisieren.

6. Verfahren nach Anspruch 1, **dadurch gekennzeichnet, dass** die Gaskammer (10) aus Materialien hergestellt ist, die geeignet sind, Innendruckwerte aufrechtzuerhalten, die niedriger als der Atmosphärendruck sind, und dass Lötschweißtechniken verwendet werden, um die Innendruckwerte aufrechtzuerhalten.

7. Verfahren nach Anspruch 1, **dadurch gekennzeichnet, dass** es ein Elektrometer mit einer variablen Vorspannung umfasst, das dazu geeignet ist, die von der Gaskammer (10) gesammelte Ladung zu messen.

## Revendications

1. Procédé de recherche et de réglage des paramètres d'une chambre à gaz pour un traitement par radiothérapie de patients cancéreux dans lequel une chambre à gaz (10) avec des électrodes plates et parallèles (7) est fournie, lesdites électrodes (7) étant placées à une distance donnée (d), une fenêtre (2) étant placée au-dessus d'une électrode (7) et des moyens isolants (4, 5, 6) étant placés au-dessous de ladite électrode (7), dans laquelle ladite chambre à gaz (10) est reliée à un collecteur (8) au moyen duquel un gaz noble, de préférence de l'argon, est inséré à l'intérieur d'une cavité (11) de ladite chambre (10) avec une pression réglable en fonction de la dose maximale par impulsion à mesurer, de sorte que le champ électrique à l'intérieur de ladite chambre (10) est dû à la polarisation de ladite chambre (10) et aux charges générées par une impulsion de rayonnement, **caractérisé en ce que** ledit procédé comprend les étapes suivantes:

- détermination d'une valeur maximale de dose par impulsion à mesurer;
- calcul de la densité de charge par unité de volume générée par l'impulsion à l'intérieur de ladite chambre à gaz (10);
- le calcul d'une valeur minimale de tension appliquée auxdites électrodes (7) nécessaire pour que le champ électrique ne s'annule jamais lors de la collecte des électrons;

- détermination d'une valeur de tension, afin de calculer la densité et la pression du gaz noble, de telle sorte que cette valeur de tension soit supérieure à la valeur de tension minimale appliquée auxdites électrodes (7);
- analyse et vérification que la paire de valeurs de densité et de tension se situe en dehors du régime Geiger et diminution éventuelle de la valeur de densité si ladite paire de valeurs se situe dans le régime Geiger, choix de la nouvelle valeur de densité afin de sortir de ce régime;
- insertion d'une dynamique temporelle des ions positifs et calcul de la densité de charge maximale présente pendant toute la durée de l'impulsion;
- détermination d'une valeur maximale acceptable d'imprécision sur la mesure, causée par la perturbation du champ électrique sur la plage de dose par impulsion à détecter, et diminution éventuelle de la valeur de la densité en cas de dépassement de cette valeur maximale.

2. Méthode selon la revendication 1 **caractérisée par le fait que**, ladite valeur de tension étant fixe, ladite pression de gaz noble est diminuée afin de sortir du régime Geiger et de garantir que le champ électrique ne s'annule jamais jusqu'à une valeur fixe de dose par impulsion.

3. Procédé selon la revendication 2, **caractérisé par le fait que** la diminution de la pression et l'augmentation de la vitesse de dérive de la charge génèrent pendant l'impulsion un effet d'appauvrissement de la charge dans la chambre à gaz (10), de sorte que la charge accumulée maximale est inférieure à la charge générée et que, par conséquent, son effet perturbateur sur le champ électrique et la production d'ionisations secondaires sont moindres.

4. Procédé selon la revendication 1, **caractérisé par** la présence, au niveau dudit collecteur (8), d'un manomètre (12) permettant de contrôler la pression (P) dudit gaz noble.

5. Procédé selon la revendication 1, **caractérisé par** la présence, au niveau dudit collecteur (8), d'un connecteur triaxial (13) adapté à la réalisation de conditions d'ultravide (UHV).

6. Procédé selon la revendication 1, **caractérisé par le fait que** ladite chambre à gaz (10) est constituée de matériaux adaptés pour maintenir des valeurs de pression interne inférieures à la pression atmosphérique et utilisant des techniques de soudure par brasage afin de maintenir lesdites valeurs de pression interne.

7. Procédé selon la revendication 1, **caractérisé en ce qu'**il comprend un électromètre ayant une tension de polarisation variable, adapté pour mesurer la charge collectée par ladite chambre à gaz (10).

Fig. 1

Fig. 2

Fig. 3B

Fig. 3A

Sez. F-F

Fig. 4

Fig. 5

Fig. 6

Fig. 7

Fig. 8

Fig. 9

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- WO 2014065990 A1

- WO 2021050535 A1

**Non-patent literature cited in the description**

- Absorbed Dose Determination in External Beam Radiotherapy. *Technical Report Series* (398)